# FASCICULE DE BREVET EUROPEEN

(11) **EP 3 975 943 B1**
(45) Date de publication et mention de la délivrance du brevet: **06.09.2023**
(21) Numéro de dépôt: 20728522.2
(22) Date de dépôt: 02.06.2020
(51) Int. Cl.: A61F 5/02

(54) **CEINTURE LOMBAIRE À CAPTEURS**
LUMBALGÜRTEL MIT SENSOREN
LUMBAR BELT WITH SENSORS

(30) Priorité: 03.06.2019 FR 1905838
(43) Date de publication de la demande: 06.04.2022
(73) Titulaire: Thuasne, 92300 Levallois Perret (FR)
(72) Inventeur: SOUCHE, Clotilde, 69007 LYON (FR); MICHEL, Philippe, 59242 CAPPELLE-EN-PEVELE (FR)
(74) Mandataire: Lavoix
(86) Numéro de dépôt international: PCT/EP2020/065214
(87) Numéro de publication internationale: WO 2020/245114

(56) Documents cités:
- US-A1- 2014 364 784
- US-B2- 9 839 553

## Description

La présente invention concerne une ceinture lombaire comprenant un corps de ceinture prévu pour entourer le tronc d'un utilisateur et au moins un capteur lié au corps de ceinture.

Pour suivre l'utilisation de la ceinture par un utilisateur et permettre un retour sur ladite utilisation, il est avantageux de prévoir des capteurs sur la ceinture.

US 9 839 553 B2 décrit une ceinture orthopédique comprenant un premier capteur de pression placé sur l'abdomen, un deuxième capteur de pression sur le dos et un capteur de détection de posture.

Cependant, le placement des capteurs par rapport à la ceinture n'est pas précis.

Or les mesures effectuées par les capteurs sont susceptibles d'être influencées par l'intégration de chaque capteur par rapport à la ceinture.

En outre, une telle ceinture ne permet pas un entretien facile.

US 2014/364784 A décrit un soutien pour torse capable d'appliquer une force au torse d'un sujet avec un délai et/ou une durée régulés, en réponse à une entrée utilisateur, des événements détectés, ou des signaux d'un dispositif de synchronisation, par exemple. Les événements détectés peuvent comprendre des modifications de la démarche, de la posture ou de l'activité du sujet.

Un but de l'invention est donc de proposer une ceinture lombaire comprenant des capteurs permettant une reproductibilité de la mesure de chaque capteur et présentant un entretien facilité.

A cet effet, l'invention a pour objet une ceinture lombaire comprenant :
- un corps de ceinture destiné à entourer au moins une partie du tronc d'un utilisateur,
- au moins un premier capteur apte à mesurer la pression exercée par la ceinture sur un utilisateur ou l'élongation du corps de ceinture, et
- un module comprenant au moins un deuxième capteur apte à mesurer au moins un paramètre relatif à l'actimétrie de l'utilisateur, le module étant fixé de façon amovible à un deuxième emplacement du corps de ceinture.

L'amovibilité du module permet de faciliter l'entretien de la ceinture.

Le placement des capteurs permet en outre une reproductibilité des mesures, de sorte que les mesures réalisées sont interprétables quant au niveau de pression exercée par la ceinture sur un utilisateur.

La ceinture lombaire peut en outre présenter une ou plusieurs des caractéristiques ci-dessous, considérée(s) individuellement ou selon toutes les combinaisons techniquement possibles :
- le module comprend l'au moins un premier capteur ;
- le module comprend un étui externe définissant un volume interne, l'au moins un premier capteur et l'au moins un deuxième capteur étant compris dans le volume interne ;
- le corps de ceinture est pourvu d'une poche, le module étant apte à être inséré dans la poche ;
- l'au moins un premier capteur est fixé sur une partie ventrale du corps de ceinture destinée à être placée contre une portion ventrale du tronc de l'utilisateur ;
- le corps de ceinture comprend au moins un fil élastique, le premier capteur étant apte à mesurer l'élongation dudit fil élastique ;
- le corps de ceinture est tissé ou tricoté, le premier capteur comprenant au moins une borne de sortie, la ceinture présentant une connexion filaire entre la borne de sortie et le module, la connexion filaire comprenant au moins un fil conducteur relié à ladite borne de sortie, le fil conducteur étant confectionné avec le corps de ceinture ;
- le corps de ceinture comprend au moins une portion élastique, le fil conducteur étant élastique et confectionné dans la portion élastique du corps de ceinture ;
- le fil conducteur est pourvu d'un vernis isolant, le fil conducteur étant raccordé au niveau d'au moins un point de raccordement, un matériau protecteur étant déposé localement au niveau de chaque point de raccordement ;
- le module comprend un contrôleur, le premier capteur et le deuxième capteur étant connectés audit contrôleur ; et/ou
- le module comprend une unité de transfert, le contrôleur étant relié à l'unité de transfert à distance de données, par exemple, un émetteur d'ondes radioélectriques.

D'autres caractéristiques et avantages de l'invention apparaîtront à la lecture de la description qui suit de modes de réalisation de l'invention, donnée à titre d'exemple uniquement et en référence aux dessins dans lesquels :
**[****Fig 1****]** la figure 1 est une vue schématique d'une ceinture lombaire selon un premier mode de réalisation de l'invention,

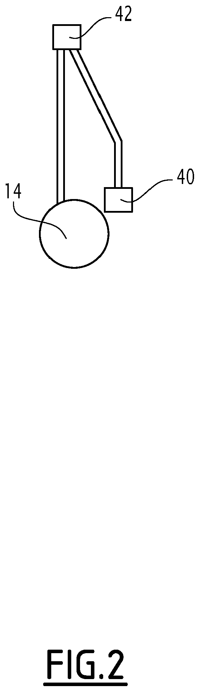

**[****Fig 2****]** la figure 2 est une vue schématique d'un premier capteur et d'un capteur additionnel de la ceinture lombaire de la figure 1,

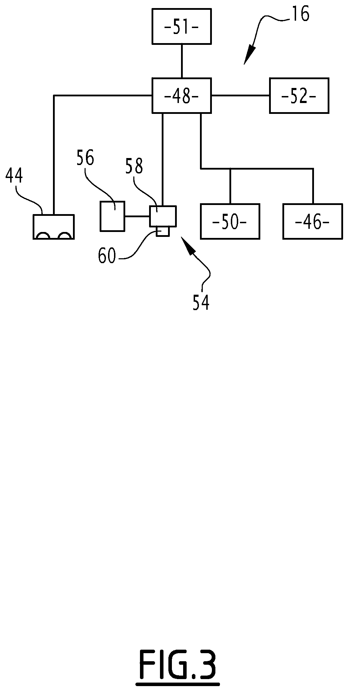

**[****Fig 3****]** la figure 3 est une vue schématique d'un module de la ceinture lombaire de la figure 1, et
[Fig 4] la figure 4 est une vue schématique d'une ceinture lombaire selon un deuxième mode de réalisation de l'invention.

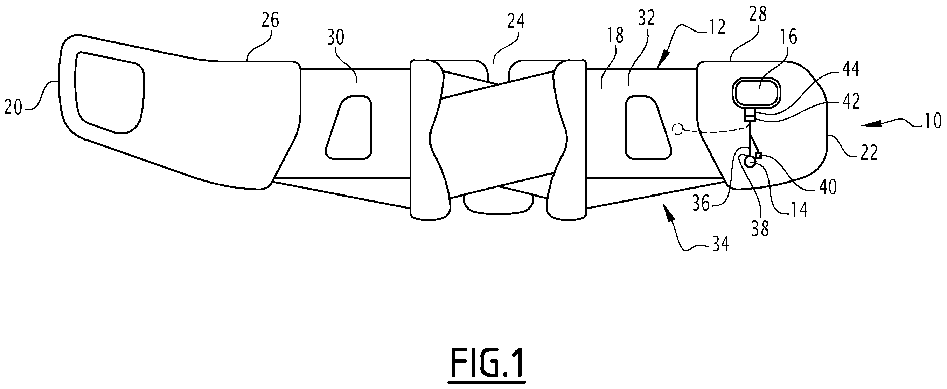

Une ceinture lombaire 10 selon un premier mode de réalisation de l'invention est représentée sur la figure 1.

La ceinture lombaire 10 comprend un corps de ceinture 12 destiné à entourer au moins une partie du tronc d'un utilisateur lorsque la ceinture 10 est portée par cet utilisateur, au moins un premier capteur 14, et un module 16.

Le corps de ceinture 12 est prévu pour appliquer une pression sur un utilisateur lorsqu'il entoure le tronc dudit utilisateur, de manière à soutenir le tronc de l'utilisateur, notamment pour prévenir ou soulager un mal de dos.

Le corps de ceinture 12 présente une face interne non représentée sur la figure 1 et destinée à être orientée vers le tronc de l'utilisateur et une face externe 18 visible sur la figure 1 et opposée à la face interne.

Le corps de ceinture 12 s'étend entre une première extrémité 20 et une deuxième extrémité 22, la première extrémité 20 et la deuxième extrémité 22 étant attachées l'une à l'autre de manière réversible lorsque le corps de ceinture 12 est porté par un utilisateur.

La première extrémité 20 et la deuxième extrémité 22 sont, par exemple, chacune pourvues d'au moins une bande autoagrippante complémentaire du type boucles et crochets, à l'une extrémité sur la face interne et à l'autre extrémité sur la face externe 18, les bandes autoagrippantes coopérant entre elles lorsque la ceinture est fermée autour du tronc de l'utilisateur.

Le corps de ceinture 12 comprend une portion dorsale 24 prévue pour s'étendre contre la colonne vertébrale d'un utilisateur lorsque la ceinture est portée, deux portions ventrales 26, 28 prévues pour s'étendre contre une portion ventrale du tronc d'un utilisateur lorsque la ceinture est portée et deux portions intermédiaires 30, 32 reliant la portion dorsale et une des portions ventrales respectives. Les première et deuxième extrémités 20 et 22 s'étendent chacune au voisinage de l'une des extrémités libres des portions ventrales.

Le corps de ceinture 12 est ici tissé ou tricoté.

Le corps de ceinture 12 comprend au moins une portion élastique 34.

On entend ici par « élastique » que le corps de ceinture 12 dans ladite portion est apte à être étiré d'au moins 10%, plus particulièrement d'au moins 20% et avantageusement compris entre 55% et 65% sans endommagement et à retrouver sa dimension initiale en l'absence d'effort d'étirement dans sa zone élastique.

La partie élastique 34 est située dans une des portions intermédiaires 32, du corps de ceinture 12.

Le corps de ceinture 12 comprend au moins un fil élastique, plus particulièrement dans la partie élastique. La partie élastique est, par exemple, formée essentiellement de fils élastiques.

Alternativement, le corps de ceinture 12 ne comprend ni partie élastique ni fil élastique.

Le premier capteur 14 est apte à mesurer la pression exercée par la ceinture 10 sur un utilisateur ou l'élongation du corps de ceinture 12, plus particulièrement de l'étirement du corps de ceinture 12 dans la portion élastique 34.

Le premier capteur 14 est, par ailleurs, étanche ou encapsulé de manière étanche dans le corps de ceinture 12 de sorte qu'il peut subir sans dommage un lavage de la ceinture.

Le premier capteur 14 comprend au moins une borne de sortie.

Dans le mode de réalisation représenté en trait plein, le premier capteur 14 est fixé sur une des portions ventrales du corps de ceinture 12.

La portion ventrale n'est ici pas élastique.

Le premier capteur 14 est, par exemple, un capteur de pression capacitif, c'est-à-dire qu'il présente une capacité qui augmente lorsqu'une pression est appliquée sur le capteur 14, ladite capacité étant mesurée pour calculer la pression correspondante.

Alternativement, le premier capteur 14 est un capteur de pression résistif.

Dans le mode de réalisation représenté en trait interrompu, le premier capteur 14 est fixé sur une portion intermédiaire 32, plus particulièrement dans la partie élastique 34.

Additionnellement ou alternativement, le premier capteur 14 est apte à mesurer l'élongation d'un fil ou d'une portion de la ceinture. On entend alors par élongation de la ceinture, l'élongation dudit fil ou de ladite portion de ceinture.

Le premier capteur 14 est, par exemple, apte à mesurer l'élongation dans la partie élastique, plus particulièrement d'un des fils élastiques formant la partie élastique.

Le premier capteur 14 est, par exemple, un capteur d'élongation inductif ou un capteur d'élongation résistif basé sur une fibre optique élastique intégrant des conducteurs tel que décrit dans WO 2017137945 A1 ou WO2014047660 A1.

Le premier capteur 14 est fixé de façon inamovible à un premier emplacement du corps de ceinture 12.

Le premier emplacement est un emplacement fixe et prédéterminé du corps de ceinture 12.

Cela permet de connaître le positionnement précis du premier capteur 14 par rapport à la ceinture, et ainsi le positionnement du premier capteur 14 par rapport à l'utilisateur lorsque celui-ci serre la ceinture avec un même niveau de serrage, ce qui assure une cohérence des mesures d'une mesure à une autre.

Cela permet notamment une interprétation des mesures du premier capteur 14 plus adaptée.

Il est, par exemple, intégré audit corps de ceinture 12.

On entend par « intégré » que le premier capteur est incorporé dans le corps de ceinture 12. Il est, par exemple, inséré dans le tissu ou le tricot formant le corps de ceinture 12. Alternativement, le premier capteur 14 n'est pas inséré dans le tissu ou le tricot, mais l'ensemble des fils de connexions dudit premier capteur 14 l'est.

Dans le mode de réalisation représenté à la figure 1, le premier capteur est intégré dans la partie élastique 34 du corps de ceinture 12. Le premier capteur 14 est ici apte à mesurer la pression exercée par la ceinture 10 sur un utilisateur ou l'élongation du corps de ceinture 12.

Dans un mode de réalisation alternatif, le premier capteur 14 est intégré dans une partie non élastique, voire rigide, de la ceinture. Le premier capteur 14 est alors apte à mesurer la pression exercée par la ceinture 10 sur l'utilisateur.

Le premier capteur 14 est, par exemple, intégré dans une platine comportant en outre un circuit intégré ou des fils prévus pour réaliser une connexion avec le module. La platine est ici étanche, plus particulièrement réalisée en matière plastique, et est intégrée dans le corps de ceinture 12.

La ceinture 10 comprend avantageusement un capteur additionnel de température 40 apte à mesurer la température du premier capteur 14 ou autour de celui-ci. Cela permet notamment d'ajuster la mesure du premier capteur 14 en fonction de la température, dans les cas où une variation de la température serait susceptible d'entraîner une dérive dans les mesures réalisées par le premier capteur 14.

Alternativement, le premier capteur 14 n'est pas sensible à la température au moins dans une gamme d'utilisation. La ceinture 10 ne comprend pas de capteur additionnel de température 40 apte à mesurer la température du premier capteur 14 ou autour de celui-ci.

La ceinture est, par exemple, associée à un identifiant unique, par exemple, mémorisé dans un support tel qu'une puce par radio-identification (RFID pour « *Radio Frequency Identification »*) ou par communication en champ proche (NFC pour « *Near Field Communication »*)*.* L'identifiant unique comprend une indication du modèle de la ceinture, le contrôleur étant apte à lire ledit identifiant au sein de la puce.

Le support de l'identifiant unique est ici solidaire du premier capteur 14.

Le module 16 est fixé de façon amovible à un deuxième emplacement du corps de ceinture 12. Le deuxième emplacement du corps de ceinture 12 est fixe sur la ceinture 10, c'est-à-dire que le module 16 est fixé au même point lorsqu'il est à nouveau fixé sur le corps de ceinture 12 après en avoir été retiré.

Le module 16 est fixé à proximité du premier capteur 14, plus particulièrement dans la partie élastique 34 du corps de ceinture 12.

Le module 16 est fixé de manière amovible à l'une des portions ventrales 28 du corps de ceinture 12.

Le module 16 est, par exemple, formé d'une coque délimitant un volume intérieur, l'ensemble des éléments du module 16 décrits ci-dessous étant compris dans le volume intérieur.

Le module 16, plus particulièrement la coque, présente une interface de connexion 44 prévue pour raccorder électriquement le module à une borne correspondante 42 du corps de ceinture 12. Ladite borne forme l'emplacement fixe de fixation du module 16.

Le module 16 comprend au moins un deuxième capteur 46 apte à mesurer au moins un paramètre relatif à l'actimétrie de l'utilisateur.

Le positionnement précis du deuxième capteur 46 est connu par rapport à la ceinture, le module 16 étant fixé à un emplacement donné, et ainsi le positionnement du deuxième capteur 46 par rapport à l'utilisateur lorsque celui-ci serre la ceinture avec un même niveau de serrage.

Cela permet, par exemple, d'interpréter très précisément les mouvements de l'utilisateur.

Le deuxième capteur 46 est, par exemple, un capteur de mouvement fonctionnant selon trois axes, c'est-à-dire apte à mesurer le mouvement selon trois axes formant un espace, ou un gyroscope.

Le paramètre relatif à l'actimétrie est par exemple un nombre de pas réalisé par l'utilisateur portant la ceinture 10.

Le module 16 comprend en outre un contrôleur 48, plus particulièrement un microprocesseur apte à réaliser les traitements de données transmis par le premier capteur 14, le deuxième capteur 46 et le capteur additionnel de température 40 par logiciel.

Le module 16 comprend en outre une mémoire 50 reliée au contrôleur 48, de sorte que le contrôleur 48 a accès à ladite mémoire 50 pour y stocker et/ou en extraire des données.

Le module 16 comprend en outre une horloge 51 associée au contrôleur 48. L'horloge 51 est adaptée pour fournir une datation à tout instant.

Le deuxième capteur 46 est relié au contrôleur 48, de manière à permettre la transmission de données depuis le deuxième capteur 14 audit contrôleur 20.

Le deuxième capteur 46 est connecté audit contrôleur 48, plus particulièrement de manière filaire directement au sein du module.

Alternativement, le deuxième capteur 46 est relié au contrôleur 48 de manière non filaire, par exemple par un moyen de communication sans fil tel que par ondes radio ou en communication en champ proche (ou NFC de « *Near Field Communication »*)*.*

Le contrôleur 48 est en outre relié à une unité de transfert 52 à distance de données.

L'unité de transfert 52 est apte à émettre un signal comprenant des données transmises par le contrôleur 48.

L'unité de transfert 52 est reliée au contrôleur 48, par exemple, par liaison filaire au sein du module 16.

L'unité de transfert 52 est, par exemple, un émetteur d'ondes radioélectriques. Elle suit, par exemple, le protocole Wi-Fi ^{™} ou la norme Bluetooth ^{®}.

Alternativement, l'unité de transfert 52 utilise la communication en champ proche (ou NFC de « *Near Field Communication »*)*.*

L'unité de transfert 52 permet notamment de transférer des données du contrôleur 48 à un ordinateur externe, par exemple, pour permettre un retour à l'utilisateur ou un suivi du patient.

Le module 16 comprend en outre un système de batterie 54 relié au contrôleur 48, avantageusement de manière filaire au sein du module 16.

Le système de batterie 54 comprend ici une batterie 56 rechargeable et un chargeur 58.

La batterie 56 est, par exemple, un accumulateur Lithium-Ion.

Le chargeur 58 comprend une interface de recharge 60 prévue pour être raccordée à une source d'électricité, comme un réseau électrique, pour recharger la batterie 56 via le chargeur 58.

L'interface de recharge 60 est, par exemple, une connexion micro-USB.

La ceinture 10 présente une connexion filaire interruptible entre la borne de sortie du premier capteur 12 et le module 16.

La connexion filaire comprend au moins un fil conducteur 36 présentant une première extrémité reliée à ladite borne de sortie et une deuxième extrémité pourvue d'une borne de connexion 42.

La borne de connexion 42 correspond à la borne de fixation du module 16 décrit précédemment.

La borne de connexion 42 est complémentaire de l'interface de connexion 44 du module 16. L'interface de connexion 44 du module 16 est fixée de manière amovible à la borne de connexion 42, de manière à former la connexion filaire entre le premier capteur et le module.

Le fil conducteur 36 est, dans le mode de réalisation représenté, élastique et intégré directement dans le tissu ou le tricot élastique du corps de ceinture, c'est-à-dire que le fil conducteur élastique 36 est tissé ou tricoté directement avec les fils constituant le corps de ceinture 12.

Alternativement, le fil conducteur 36 est cousu, brodé ou ajouté au corps de ceinture après la réalisation du corps de ceinture 12.

On dit que le fil conducteur 36 est confectionné avec le corps de ceinture 12.

Alternativement, le fil conducteur 36 n'est pas élastique.

Le fil conducteur élastique 36 présente une résistance comprise entre 5 ohms et 20 ohms par mètre de fil.

Le fil conducteur élastique 36 présente un titre global compris entre 730 dtex et 2460 dtex. Cela permet notamment de tricoter ou tisser le fil conducteur élastique avec le corps de ceinture 12 sur une machine standard.

Le fil conducteur élastique 36 comprend, par exemple, un fil d'âme élastique, au moins un fil guipé conducteur d'électricité enroulé par guipage autour du fil d'âme élastique et au moins un fil non conducteur recouvrant le fil guipé conducteur.

Le fil non conducteur recouvre, par exemple, entièrement le fil guipé conducteur.

Le fil non conducteur est également guipé autour du fil d'âme élastique, plus particulièrement selon le sens inverse du fil guipé conducteur.

Le fil d'âme élastique présente un double guipage.

Le fil guipé conducteur est composé de cuivre et/ou d'argent. Plus particulièrement, il s'agit d'un fil de cuivre entouré d'une couche d'argent.

Le fil guipé conducteur présente un diamètre compris entre 30 µm et 100 µm. Il comporte un ou plusieurs filaments conducteurs.

Le fil guipé conducteur est avantageusement verni à l'aide d'un vernis isolant. Cela permet notamment de rendre le fil guipé conducteur étanche, de le protéger de dégradations physiques ou d'oxydation.

Le fil guipé conducteur présente un nombre de tours par mètre compris entre 600 et 1700. Cela permet notamment une conduction de l'électricité optimale.

Le fil guipé conducteur présente un allongement à la rupture strictement supérieur à 60%, ici plus particulièrement strictement supérieur à 80%.

Le fil conducteur élastique 36 est raccordé au niveau de point(s) de raccordement 38, notamment à la borne de sortie du premier capteur 14 et à la borne de connexion 42. A chaque point de raccordement, le fil conducteur est dépourvu de vernis, un matériau protecteur étant déposé localement au niveau de chaque point de raccordement. Le matériau protecteur est, par exemple, du silicone.

Cela permet notamment de rendre étanche l'ensemble au niveau des points de raccordement. Cela est avantageux pour permettre un lavage de la ceinture, sans endommager les connexions électriques.

Avantageusement, le capteur additionnel de température 40 est également relié à la borne de connexion 42, avantageusement par un fil conducteur élastique similaire à celui reliant le premier capteur à la borne de connexion 42, de manière à être fixé de manière réversible à l'interface de connexion du module 18.

Ainsi, le premier capteur 14 et le capteur additionnel de température 40 sont chacun reliés au contrôleur 48 du module 16, de manière à permettre la transmission de données depuis chacun desdits capteurs 14, 40 audit contrôleur 48, plus particulièrement via l'interface de connexion 44 du module 16.

Alternativement, le premier capteur 14 et/ou le capteur additionnel de température 40 sont reliés au contrôleur 48 de manière non filaire, par exemple par un moyen de communication sans fil tel que par ondes radio ou en communication en champ proche (ou NFC de « *Near Field Communication »*)*.*

L'intégration du premier capteur 14 dans la ceinture 10 et la structure prévue pour le raccordement du module 16 permettent notamment de rendre le corps de ceinture 12 avec le premier capteur 14 intégré lavable en machine. Par exemple, le corps de ceinture 12 avec le premier capteur 14 résiste à un nombre de lavage à une température de 30°C strictement supérieur à 15.

Le placement du deuxième capteur dans un module amovible permet en outre de faciliter l'entretien de la ceinture. En effet, il suffit de retirer le module avant le lavage de la ceinture. Ainsi, il n'y a pas de risque d'endommager le module et le deuxième capteur placé à l'intérieur pendant le lavage de la ceinture.

Dans un mode de réalisation non représenté, la ceinture lombaire comprend en outre une bande additionnelle prévue pour entourer le tronc d'un utilisateur autour du corps de ceinture 12 de manière à appliquer une compression supplémentaire.

L'intégration du premier capteur est sur ou au sein du corps de ceinture permet un placement fixe dudit capteur.

Les mesures réalisées sont reproductibles, de sorte que celles-ci sont interprétables quant au niveau de serrage de la ceinture sur l'utilisateur.

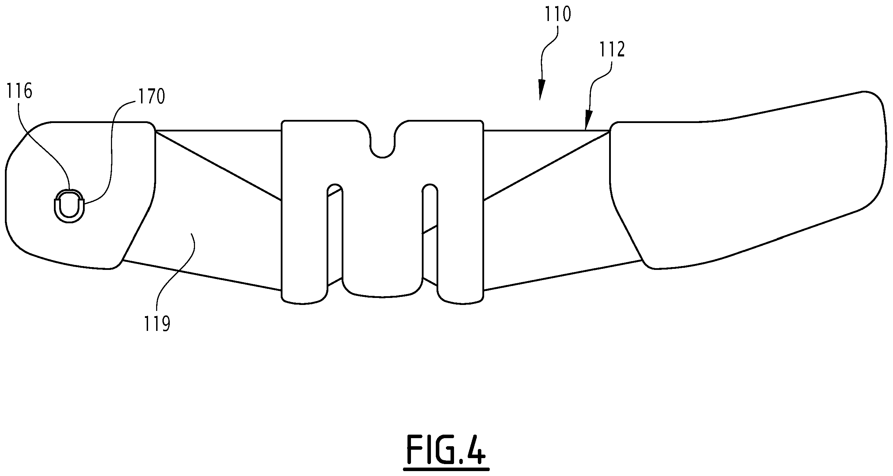

Une ceinture 110 selon un deuxième mode de réalisation est représentée sur la figure 4. Seules les caractéristiques par lesquels le deuxième mode de réalisation diffère du premier mode de réalisation sont décrites ici.

Les éléments identiques ou similaires présentent une référence égale à celle du premier mode de réalisation incrémentée de 100.

Le corps de ceinture 112 est pourvu d'une poche 170.

La poche 170 s'étend sur la face interne 119 du corps de ceinture 112.

La poche 170 comprend un textile, ici un tissu, définissant un volume interne de la poche 170.

La poche 170 présente une unique ouverture permettant l'accès audit volume interne de la poche sur un bord supérieur du textile.

Le périmètre du textile en dehors du bord supérieur est solidaire du, par exemple cousu au, corps de ceinture 112.

Le périmètre comprend un bord inférieur, le bord supérieur et deux côtés reliant chacun le bord inférieur et le bord supérieur.

La poche 170 est avantageusement pourvue d'une languette fixée au textile. La languette facilite la prise du textile de manière à écarter le textile du corps de ceinture 112 de manière à faciliter l'accès au volume interne de la poche.

Le textile présente une élasticité comprise entre 20 % et 200 %. Il est, par exemple, réalisé en Lycra.

Alternativement, le textile n'est pas élastique. La poche définit un volume interne complémentaire du module 116.

Dans ce deuxième mode de réalisation, le premier capteur n'est pas fixé sur une des portions ventrales du corps de ceinture, mais est intégré au module 116.

Le premier capteur est, par exemple, un capteur de pression capacitif ou résistif.

Le module 116 comprend un étui externe définissant un volume interne, l'au moins un premier capteur et l'au moins un deuxième capteur étant compris dans le volume interne.

Le contrôleur, la mémoire, l'unité de transfert et le système de batterie tels que décrit en regard du premier mode de réalisation sont similairement compris dans l'étui externe du module 116.

Le premier capteur est ici directement relié au contrôleur 48 par connexion filaire au sein du module 116.

Le module 116, plus particulièrement l'étui externe, ne présente pas d'interface de connexion prévue pour raccorder électriquement le module à une borne correspondante du corps de ceinture.

L'ensemble des éléments électroniques est compris dans le module 116.

L'intégration de l'ensemble de l'électronique dans le module amovible 116 permet notamment de rendre le corps de ceinture 112 lavable en machine en retirant simplement le module 116.

Le module 116 est apte à être inséré, ici par le bord supérieur, de façon amovible dans la poche 170, plus particulièrement dans le volume interne de la poche 170, correspondant ici au deuxième emplacement du corps de ceinture 12.

La poche 170 est dimensionnée telle que le module 116 inséré dans la poche 170 soit maintenu fixe par rapport au corps de ceinture 112 par le textile de la poche 170.

Plus particulièrement, le module 116 est prévu pour être inséré dans la poche 170 avec un jeu inférieur à 0,1 mm, de préférence sans jeu.

En outre, l'élasticité du textile améliore le maintien du module 116 par la poche 170 contre le corps de ceinture 112, plus particulièrement contre la face interne 119.

Ainsi, le deuxième emplacement du corps de ceinture 112 est fixe sur la ceinture 110.

Le positionnement précis du module 116 dans la poche 170 permet un positionnement précis du premier capteur et du deuxième capteur.

Cela permet de connaître le positionnement précis du premier capteur et du deuxième capteur par rapport à la ceinture, et ainsi le positionnement du premier capteur et du deuxième capteur par rapport à l'utilisateur lorsque celui-ci serre la ceinture avec un même niveau de serrage, ce qui assure une cohérence des mesures d'une mesure à une autre.

Cela permet notamment une interprétation des mesures du premier capteur et du deuxième capteur plus adaptée.

Les mesures réalisées sont reproductibles, de sorte que celles-ci sont interprétables.

Dans un mode de réalisation alternatif semblable au premier mode de réalisation, le module 16 décrit en relation avec le premier mode de réalisation est fixé à la ceinture par insertion dans une poche telle que décrit en regard du deuxième mode de réalisation.

Une ceinture lombaire selon l'invention permet donc une reproductibilité de la mesure de chaque capteur et est facile d'entretien.

Une telle ceinture lombaire 10, 110 permet notamment un suivi du port de la ceinture, notamment via un procédé de détermination du port de la ceinture par un utilisateur.

Un exemple de mode de réalisation du procédé comprend les étapes suivantes :
- fourniture d'une ceinture telle que décrite précédemment,
- acquisition de premières données par l'au moins un premier capteur 14,
- acquisition de deuxièmes données par l'au moins un deuxième capteur 46, et
- traitement simultané des premières données et des deuxièmes données pour déterminer si la ceinture est portée.

Au cours de l'étape d'acquisition des premières données, l'au moins un premier capteur 14 acquiert des premières données correspondant à la pression appliquée par l'orthèse sur un utilisateur ou à l'élongation de l'orthèse, comme décrit précédemment.

Les mesures sont prises par le premier capteur 14 à intervalle régulier.

Alternativement, le premier capteur 14 acquière des mesures en continu.

Les premières données sont ensuite transmises au contrôleur 18.

Les premières données sont susceptibles de subir un ou des prétraitements de données avant ou pendant la transmission. Dans ce cas, on entend par les premières données transmises, les premières données prétraitées et transmises.

Alternativement, le ou les prétraitements sont réalisés après la transmission par le contrôleur 48.

Le ou les prétraitements correspondent, par exemple, à un débruitage notamment par filtrage fréquentiel.

Au cours de l'étape d'acquisition des deuxièmes données, l'au moins un deuxième capteur 46 acquiert des deuxièmes données correspondant à au moins un paramètre relatif à l'actimétrie de l'utilisateur, comme décrit précédemment.

Les mesures sont prises par le deuxième capteur 46 à intervalle régulier, par exemple, avec une fréquence de 25 Hz.

Alternativement, le deuxième capteur 46 acquière des mesures en continu.

Les deuxièmes données sont ensuite transmises au contrôleur 48.

Les deuxièmes données sont susceptibles de subir un ou des prétraitements de données avant ou pendant la transmission. Dans ce cas, on entend par les deuxièmes données transmises, les deuxièmes données prétraitées et transmises.

Alternativement, le ou les prétraitements sont réalisés après la transmission par le contrôleur 48.

Le ou les prétraitements correspondent, par exemple, à un débruitage notamment par filtrage fréquentiel.

Les premières et deuxièmes données transmises sont ensuite traitées.

L'étape de traitement simultané des données est ici réalisée par le contrôleur 48.

Elle prend, par exemple, en compte le modèle de ceinture pour permettre une détection plus fiable des données par les capteurs par rapport à la ceinture.

Le contrôleur 48 considère, par exemple, initialement que l'orthèse n'est pas portée.

Si une première condition de port et une deuxième condition de port sont remplies de manière concomitante, alors il est déterminé que la ceinture est portée.

La première condition de port est ici que pendant une première période supérieure à une première période minimale, la pression ou l'élongation mesurée par le premier capteur est supérieure à une première valeur prédéterminée MinP.

La première valeur prédéterminée MinP correspond ici à une pression minimale ou à une élongation minimale.

La deuxième condition de port est ici que pendant une deuxième période supérieure à une deuxième période minimale Tobs, le paramètre relatif à l'actimétrie est supérieur à une deuxième valeur prédéterminée.

La deuxième valeur prédéterminée correspond à une valeur minimale de mouvement sur chacun des axes.

La première période est incluse dans la deuxième période ou la deuxième période est incluse dans la première période.

Ici la première période et la deuxième période coïncident.

Lorsque le port de l'orthèse est détecté alors que précédemment l'orthèse était indiquée comme non portée, le contrôleur enregistre un évènement correspondant au début de port de l'orthèse auquel une datation fournie par l'horloge interne est associée.

Par ailleurs, si, lors de l'étape de traitement simultané des données, il est déterminé que la ceinture est portée, alors le procédé comprend, en outre, une étape de détermination d'un niveau de serrage de l'orthèse.

Lorsque l'orthèse est précédemment détectée portée, mais que l'une de première et deuxième conditions de retrait est réalisée, alors il est détectée que l'orthèse n'est plus portée.

La première condition est ici que pendant une première période supérieure à une première période minimale, la pression ou l'élongation mesurée par le premier capteur est inférieure à une première valeur prédéterminée MinP.

La première période et la première valeur prédéterminée MinP sont ici identiques à ceux de la première condition.

La deuxième condition est ici que pendant une deuxième période supérieure à une deuxième période minimale Tobs, le paramètre relatif à l'actimétrie est inférieur à une deuxième valeur prédéterminée.

La deuxième période Tobs et la deuxième valeur prédéterminée sont ici identiques à ceux de la première condition.

Le contrôleur enregistre alors un évènement correspondant à la fin de port de la ceinture auquel une datation fournie par l'horloge interne est associée.

Suite à l'étape de traitement, le procédé comprend avantageusement une étape de transmission d'au moins un résultat de l'étape de traitement simultané des données du contrôleur du module 16 à un ordinateur extérieur par l'unité de transfert 52.

L'au moins un résultat transmis à l'ordinateur extérieur comprend le niveau de serrage.

L'ordinateur extérieur est apte à afficher l'au moins un résultat transmis pour permettre un suivi, par exemple, pour l'utilisateur et/ou pour un praticien médical suivant ledit utilisateur.

Avantageusement, l'ordinateur extérieur calcule des informations supplémentaires à partir de l'au moins un résultat transmis par l'unité de transfert 52, notamment le temps de port de la ceinture.

L'utilisation de deux types de capteurs et le traitement simultané des données permettent une meilleure fiabilité de détection du port de l'orthèse en croisant les données obtenues, ainsi qu'avantageusement de données propres au modèle de la ceinture, et en détectant de manière plus fiable la situation à un instant donné.

Cela permet notamment d'avoir de meilleures données pour le suivi médical d'un patient ou un meilleur retour à un utilisateur.

La répétition des mesures permet notamment de vérifier l'observance d'un traitement. Par ailleurs, les données obtenues par la ceinture peuvent être liées à d'autres informations, telles que la douleur ou la gêne ressentie par le patient, pour savoir si la ceinture est efficace.

## Revendications

1. Ceinture lombaire (10 ; 110) comprenant :
- un corps de ceinture (12 ; 112) destiné à entourer au moins une partie du tronc d'un utilisateur,
- au moins un premier capteur (14) apte à mesurer la pression exercée par la ceinture (10 ; 110) sur un utilisateur ou l'élongation du corps de ceinture (12 ; 112), et
- au moins un deuxième capteur (46) apte à mesurer au moins un paramètre relatif à l'actimétrie de l'utilisateur,
**caractérisé en ce que** la ceinture lombaire (10 ; 110) comprend un module (16 ; 116) comprenant l'au moins un deuxième capteur (46), le module (16 ; 116) étant fixé de façon amovible à un deuxième emplacement du corps de ceinture (12 ; 112).

2. Ceinture lombaire selon la revendication 1, dans laquelle le module (16 ; 116) comprend l'au moins un premier capteur (14).

3. Ceinture lombaire selon la revendication 2, dans laquelle le module (16 ; 116) comprend un étui externe définissant un volume interne, l'au moins un premier capteur (14) et l'au moins un deuxième capteur (46) étant compris dans le volume interne.

4. Ceinture lombaire selon l'une quelconque des revendications 1 à 3, dans laquelle le corps de ceinture (12 ; 112) est pourvu d'une poche (170), le module (16 ; 116) étant apte à être inséré dans la poche.

5. Ceinture lombaire selon l'une quelconque des revendications 1 à 4, dans laquelle l'au moins un premier capteur (14) est fixé sur une partie ventrale (26, 28) du corps de ceinture (12 ; 112) destinée à être placée contre une portion ventrale du tronc de l'utilisateur.

6. Ceinture lombaire selon l'une quelconque des revendications 1 à 5, dans laquelle le corps de ceinture (12) comprend au moins un fil élastique, le premier capteur (14) étant apte à mesurer l'élongation dudit fil élastique.

7. Ceinture lombaire selon l'une quelconque des revendications 1 à 6, dans laquelle le corps de ceinture (12) est tissé ou tricoté, le premier capteur (14) comprenant au moins une borne de sortie, la ceinture (10) présentant une connexion filaire entre la borne de sortie et le module (16), la connexion filaire comprenant au moins un fil conducteur (36) relié à ladite borne de sortie, le fil conducteur étant confectionné avec le corps de ceinture (12).

8. Ceinture lombaire selon la revendication 7, dans laquelle le corps de ceinture (12) comprend au moins une portion élastique (34), le fil conducteur (36) étant élastique et confectionné dans la portion élastique (34) du corps de ceinture (12).

9. Ceinture lombaire selon la revendication 7 ou 8, dans laquelle le fil conducteur (36) est pourvu d'un vernis isolant, le fil conducteur (36) étant raccordé au niveau d'au moins un point de raccordement (38), un matériau protecteur étant déposé localement au niveau de chaque point de raccordement (38).

10. Ceinture lombaire selon l'une quelconque des revendications 1 à 9, dans laquelle le module (16 ; 116) comprend un contrôleur (48), le premier capteur (14) et le deuxième capteur (46) étant connectés audit contrôleur (48).

11. Ceinture lombaire selon la revendication 10, dans laquelle le module (16 ; 116) comprend une unité de transfert (52), le contrôleur (48) étant relié à l'unité de transfert (52) à distance de données, par exemple, un émetteur d'ondes radioélectriques.

## Patentansprüche

1. Lumbalgürtel (10 ; 110), umfassend:
- einen Gürtelkörper (12; 112), der dazu bestimmt ist, mindestens einen Teil des Rumpfs eines Benutzers zu umgeben,
- mindestens einen ersten Sensor (14), der geeignet ist, um den von dem Gürtel (10; 110) auf einen Benutzer ausgeübten Druck oder die Dehnung des Gürtelkörpers (12; 112) zu messen, und
- mindestens einen zweiten Sensor (46), der geeignet ist, um mindestens einen Parameter zu messen, der sich auf die Aktimetrie des Nutzers bezieht,
**dadurch gekennzeichnet, dass** der Lumbalgürtel (10; 110) ein Modul (16; 116) umfasst, umfassend den mindestens einen zweiten Sensor (46), wobei das Modul (16; 116) abnehmbar an einer zweiten Stelle des Gürtelkörpers (12; 112) befestigt ist.

2. Lumbalgürtel nach Anspruch 1, wobei das Modul (16; 116) den mindestens einen ersten Sensor (14) umfasst.

3. Lumbalgürtel nach Anspruch 2, wobei das Modul (16; 116) eine äußere Hülle umfasst, die ein Innenvolumen definiert, wobei der mindestens einen ersten Sensor (14) und der mindestens einen zweiten Sensor (46) in dem Innenvolumen enthalten sind.

4. Lumbalgürtel nach einem der Ansprüche 1 bis 3, wobei der Gürtelkörper (12; 112) mit einer Tasche (170) versehen ist, wobei das Modul (16; 116) geeignet ist, um in die Tasche eingeführt zu werden.

5. Lumbalgürtel nach einem der Ansprüche 1 bis 4, wobei der mindestens einen ersten Sensor (14) an einem Bauchteil (26, 28) des Gürtelkörpers (12; 112) befestigt ist, der dazu bestimmt ist, an einem Bauchabschnitt des Rumpfs des Benutzers platziert zu werden.

6. Lumbalgürtel nach einem der Ansprüche 1 bis 5, wobei der Gürtelkörper (12) mindestens einen elastischen Faden umfasst, wobei der erste Sensor (14) geeignet ist, um die Dehnung des elastischen Fadens zu messen.

7. Lumbalgürtel nach einem der Ansprüche 1 bis 6, wobei der Gürtelkörper (12) gewebt oder gestrickt ist und der erste Sensor (14) mindestens einen Ausgangsanschluss umfasst, wobei der Gürtel (10) eine verdrahtete Verbindung zwischen dem Ausgangsanschluss und dem Modul (16) aufweist, wobei die verdrahtete Verbindung mindestens einen leitenden Draht (36) umfasst, der mit dem Ausgangsanschluss verbunden ist, wobei der leitende Draht mit dem Gürtelkörper (12) konfektioniert ist.

8. Lumbalgürtel nach Anspruch 7, wobei der Gürtelkörper (12) mindestens einen elastischen Abschnitt (34) aufweist, wobei der Leitungsdraht (36) elastisch ist und in dem elastischen Abschnitt (34) des Gürtelkörpers (12) konfektioniert ist.

9. Lumbalgürtel nach Anspruch 7 oder 8, wobei der Leiterdraht (36) mit einem Isolierlack versehen ist, wobei der Leiterdraht (36) mit mindestens einem Verbindungspunkt (38) verbunden ist, wobei an jedem Verbindungspunkt (38) lokal ein Schutzmaterial aufgebracht ist.

10. Lumbalgürtel nach einem der Ansprüche 1 bis 9, wobei das Modul (16; 116) eine Steuerung (48) umfasst, wobei der erste Sensor (14) und der zweite Sensor (46) mit der Steuerung (48) verbunden sind.

11. Lumbalgürtel nach Anspruch 10, wobei das Modul (16; 116) eine Übertragungseinheit (52) umfasst, wobei die Steuereinheit (48) mit der Datenfernübertragungseinheit (52) verbunden ist, z. B. einem Funkwellensender.

## Claims

1. A lumbar support belt (10; 110) comprising:
- a support belt body (12; 112) for surrounding at least one part of a user's torso,
- at least one first sensor (14) capable of measuring the pressure exerted by the belt (10; 110) on a user or the elongation of the support belt body (12; 112), and
- at least one second sensor (46) adapted to measure at least one parameter relating to the actimetry of the user,
**characterized in that** the lumbar support belt (10; 110) comprises a module (16; 116) comprising the at least one second sensor (46), the module (16; 116) being removably attached to a second location of the support belt body (12; 112).

2. The lumbar support belt according to claim 1, wherein the module (16; 116) comprises the at least one first sensor (14).

3. The lumbar support belt according to claim 2, wherein the module (16; 116) comprises an outer casing defining an inner volume, the at least one first sensor (14) and the at least one second sensor (46) being contained in the inner volume.

4. The lumbar support belt according to any one of claims 1 to 3, wherein the support belt body (12; 112) is provided with a pocket (170), the module (16; 116) being capable of being inserted into the pocket.

5. The lumbar support belt according to any one of claims 1 to 4, wherein the at least one first sensor (14) is attached to a ventral portion (26, 28) of the support belt body (12; 112) for placement against a ventral portion of the user's torso.

6. The lumbar support belt according to any one of claims 1 to 5, wherein the support belt body (12) comprises at least one elastic thread, the first sensor (14) being capable of measuring the elongation of said elastic thread.

7. The lumbar support belt according to any one of claims 1 to 6, wherein the support belt body (12) is woven or knitted, the first sensor (14) comprising at least one output terminal, the belt (10) having a wire connection between the output terminal and the module (16), the wire connection comprising at least one conductive wire (36) connected to said output terminal, the conductive wire being woven or knitted in the support belt body (12).

8. The lumbar support belt according to claim 7, wherein the support belt body (12) comprises at least one elastic portion (34), the conductive wire (36) being elastic and woven or knitted into the elastic portion (34) of the support belt body (12).

9. The lumbar support belt according to claim 7 or 8, wherein the conductive wire (36) is provided with an insulating varnish, the conductive wire (36) being connected at at least one connection point (38), a protective material being deposited locally at each connection point (38).

10. The lumbar support belt according to any one of claims 1 to 9, wherein the module (16; 116) comprises a controller (48), the first sensor (14) and the second sensor (46) being connected to said controller (48).

11. The lumbar support belt according to claim 10, wherein the module (16; 116) comprises a transfer unit (52), the controller (48) being connected to the transfer unit (52) via remote data, such as a radio wave transmitter.
